# EUROPEAN PATENT SPECIFICATION

(11) **EP 2 293 127 B1**
(45) Date of publication and mention of the grant of the patent: **15.10.2014**
(21) Application number: 10180567.9
(22) Date of filing: 27.07.2004
(51) Int. Cl.: G02B 6/36, A61B 18/22, A61F 9/008

(54) **Ferrule connector for use with an illumination or laser source**
Ferrulestecker zur Verwendung mit einer Beleuchtungs- oder Laserquelle
Connecteur d'embout pour une utilisation avec un éclairage ou une source laser

(30) Priority: 28.07.2003 US 490399 P; 05.03.2004 US 550979 P; 05.06.2004 US 577740 P; 05.06.2004 US 577618 P; 27.07.2004 US 900939
(43) Date of publication of application: 09.03.2011
(62) Divisional of application: 04786120.8
(73) Proprietor: Synergetics, Inc., O'Fallon, MO 63368 (US)
(72) Inventor: Auld, Michael D., Flemington NJ 08822 (US); Easley, James C., O'Fallon, MO 63368 (US); Kane, Jonathan S., Hudson, NH 03051 (US); Scheller, Gregg, Wildwood, MO 63038 (US)
(74) Representative: Hano, Christian

(56) References cited:
- EP-A2- 0 940 700
- US-A- 4 697 870

## Description

### BACKGROUND OF THE INVENTION

The art of the present invention relates to fiberoptic endoscopic probes for vitreoretinal surgery in general and more particularly to a unique fiber optic connector ferrule which uniquely indicates whether the fiber is designed, best suited, or desired for illumination or laser transmission light or both.

Prior art vitreoretinal surgical procedure utilizes discrete and separate optical fibers for the delivery of typically non-coherent light for illumination and coherent laser beam light for surgical treatment of tissues. Although prior art "illuminated laser probes" of various configurations have been developed, they all utilize separate optical fiber or fibers for the non-coherent illumination stream and the coherent laser delivery. The aforesaid fibers are typically arranged side by side inside of a common needle lumen. An embodiment of this prior art technology is found in US 5 323 766 A. This prior art technology requires a larger or more than one incision in order to introduce illumination and laser treatment light into the eye or other structure, thereby generating greater trauma to the surgical site.

Prior art devices typically utilize a laser deliver core optical fiber diameter of typically 200 to 300 µm since said diameter provides the surgical laser bum spot size most commonly desired by the surgeon. The aforesaid prior art devices have been unable to provide sufficient surgically useful illumination (non-coherent white light) power through such a small fiber, primarily due to the prior art's inability to focus said non-coherent surgically useful light onto such a small spot size. Moreover, none of the prior art devices have combined the aforesaid surgically useful illumination and laser treatment light and transmitted through a single fiber, especially of the aforesaid small size.

Prior art illumination light sources typically require a minimum aggregate optical fiber core area equivalent to a fiber diameter of approximately 500 µm in order to deliver sufficient illuminating light to be considered useful by the surgeon. A fundamental prior art limitation with utilization of smaller light fibers for illumination is the size of the focus spot in the light source itself.

Ophthalmic surgical illumination devices for use with optical fibers are found in the prior art and have been manufactured by numerous companies for years. One of many such devices is described in U.S. Patent #4,757,426 issued to Scheller, et al. on July 12, 1988, Entitled *"Illumination System for Fiber Optic Lighting Instruments".* One of the most widely used illumination devices is the "Millennium" which is manufactured by Bausch and Lob®. Other manufacturers are Alcon® with the "Accurus" and Grieshaber® with the "GLS 150".

EP 0 940 700 A2 discloses a fiber optic connector used in the telecommunication industry comprising a rear body having a collet-like clamp for engaging the jacket and strength member of an engaged cable. The clamp includes a threaded sleeve forming deformable fingers between separating slots. The fingers have tapered ends which include an undersurface from which project thin backwardly tapered teeth. In order to grasp a cable jacket a camming nut is screwed on the tapered ends for contracting the collet.

US 4 697 870 A discloses a termination for fiberoptic cables comprising a ferrule having internal threads capable of threading onto threads of a fitting. On the side of the threads opposite a first section a collar is provided which extends radially beyond the threads. On the opposite side of the collar from the threads a third section is provided which includes an end designed to be plugged into an instrument or light source. A peripheral groove is formed in this end for detention.

The object of the present invention is to provide a coaxial illuminated laser endoscopic probe and active numerical aperture control apparatus and method of use which provides a unique ferrule or connector for optical fiber connection which uniquely indicates to the aforesaid apparatus source whether the optical fiber is designed, best suited, or desired for illumination or laser transmission light or both.

This object is achieved by a ferrule for use with an illumination or laser source comprising the features of claim 1. A preferred embodiment is claimed in claim 2.

The above object is further achieved by a method of utilizing a ferule connector comprising the features of claim 3.

The ferrule connector of the invention is uniquely configured to provide the precise positioning required while reducing cost. A precise connector end is combined with an integral retention thread to reduce parts cost and assembly time. An optional groove or recess is placed on the connector to provide for sensing the difference between illumination only and laser compatible output fibers. Placement of a smooth diameter connector into the output activates a switch which will allow the laser power to be mixed. Either the lack of a connector or the groove under the switch will cause the switch to not activate and the laser power will not be mixed in.

The art of the present invention comprises a ferrule or connector having an internal bore, preferably stepped, which is substantially parallel with the lengthwise axis of the ferrule body. The aforesaid bore allows for placement and bonding or potting of an optical fiber within and through said ferrule body. Externally, said ferrule body is also stepped in a unique form in order to optimally function as described herein.

Where provided herein, dimensions, geometrical attributes, and thread sizes are for preferred embodiment informational and enablement purposes. Alternative embodiments may utilize a plurality of variations of the aforesaid without departing from the scope and spirit of the present invention. The art of the present invention may be manufactured from a plurality of materials, including but not limited to metals, plastics, glass, ceramics, or composites.

### BRIEF DESCRIPTION OF THE DRAWINGS

Numerous other objects, features, and advantages of the invention should now become apparent upon a reading of the following detailed description taken in conjunction with the accompanying drawings, in which:

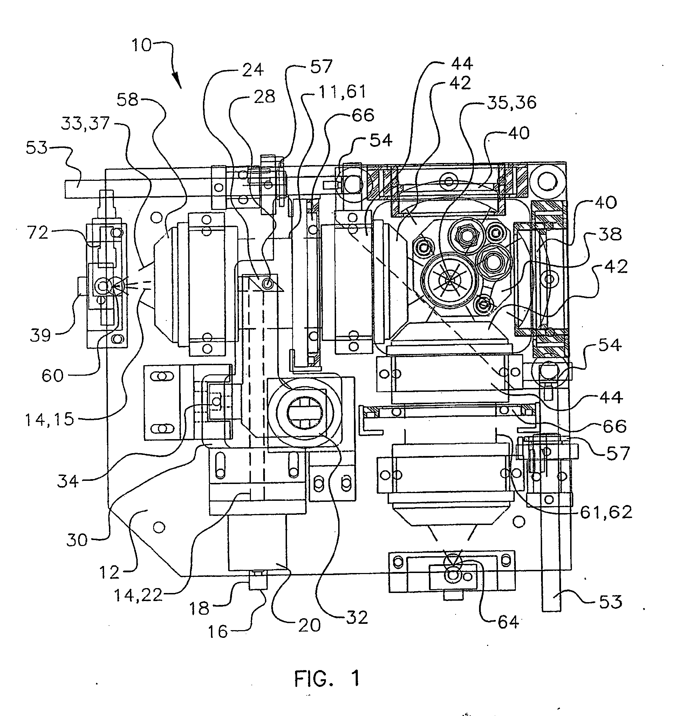

Fig. 1 is a top plan view of a coaxial illuminated laser endoscopic probe and active numerical aperture control apparatus showing illumination and laser light paths without the phototoxicity card, power meter, and ferrule connectors.

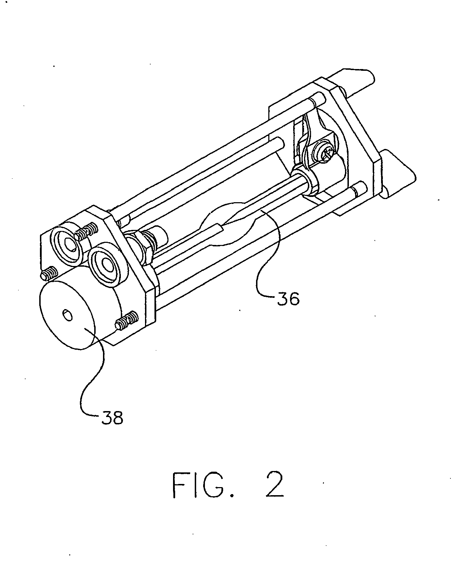

Fig. 2 is a perspective view of an arc lamp source and mount.

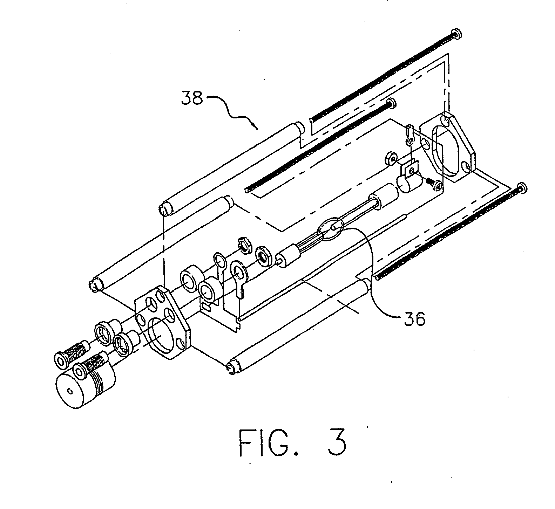

Fig. 3 is an assembly view of the arc lamp source and mount.

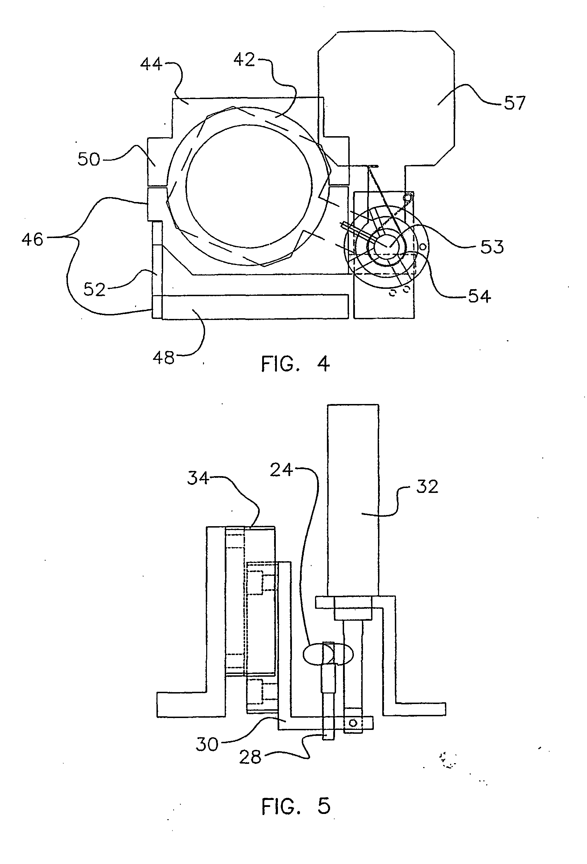

Fig. 4 is a front side plan view of a first lens mount, shaft mounted cam, and shutter with a closed position shutter shown in phantom.
Fig. 5 is a front side plan view of a steering mirror, post, bracket, ball slide, and solenoid in a non-energized extended position.

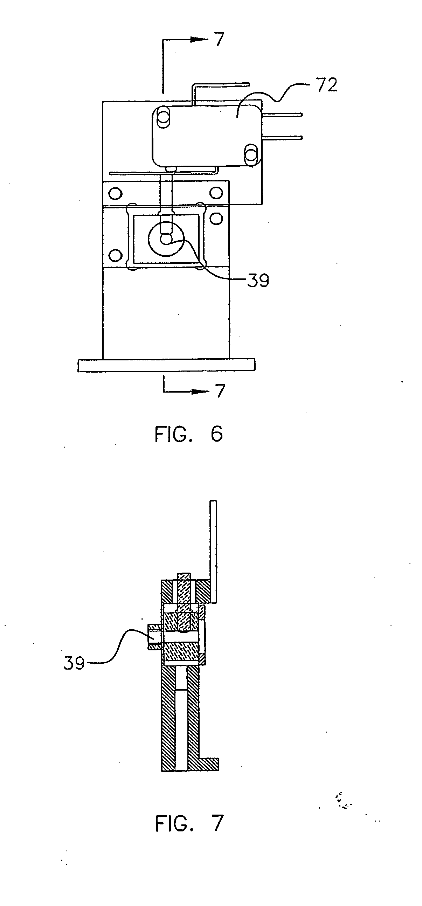

Fig. 6 is a front side plan view of a first output for laser and illumination light and the switch for sensing the recess in the alignment barrel.
Fig. 7 is a cross sectional view taken along line 7 - 7 of Fig. 6 without a switch body attached.

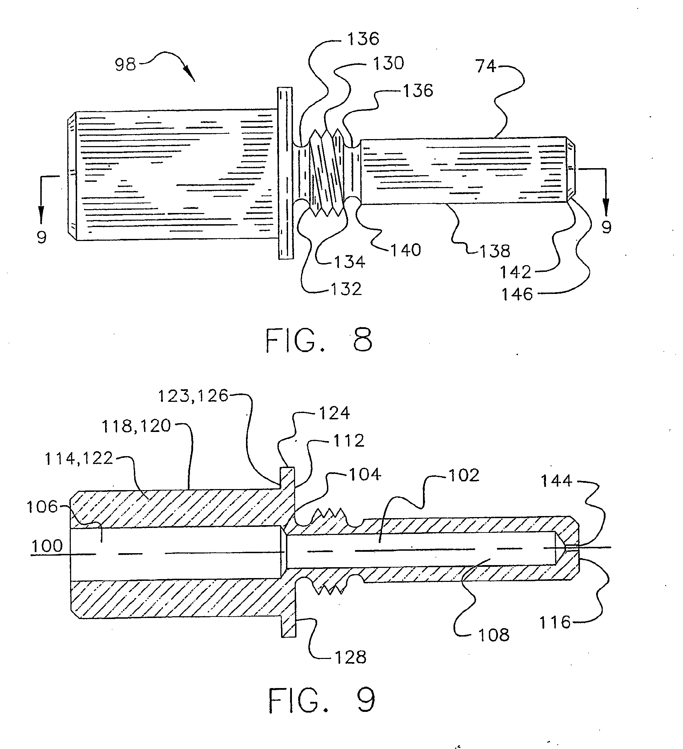

Fig. 8 is a side plan view of a ferrule connector without a recess for preferably laser and illumination use.
Fig. 9 is a cross sectional view taken along line 9 - 9 of Fig. 8.

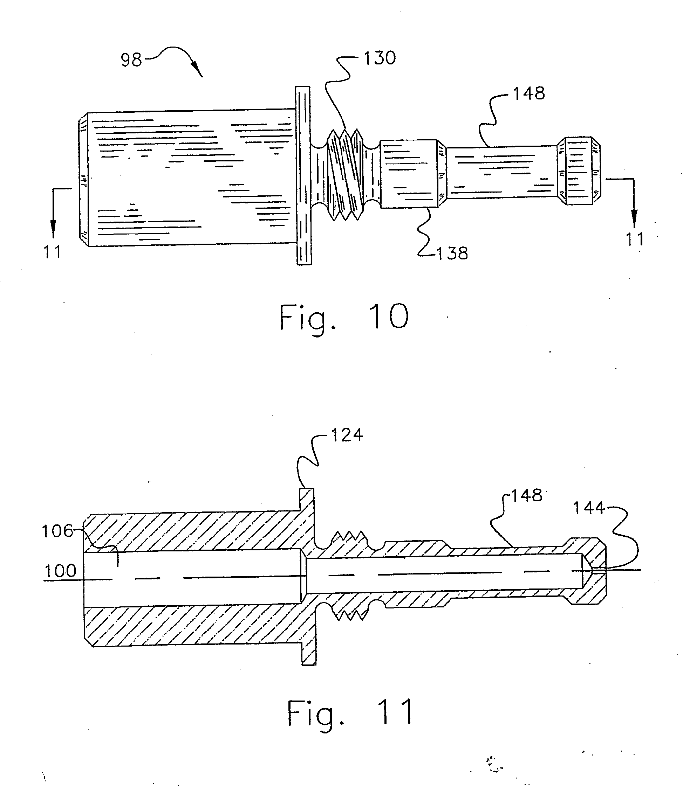

Fig. 10 is a side plan view of a ferrule connector according to the invention with a recess for preferably illumination use.
Fig. 11 is a cross sectional view taken along line 11 - 11 of Fig. 10.

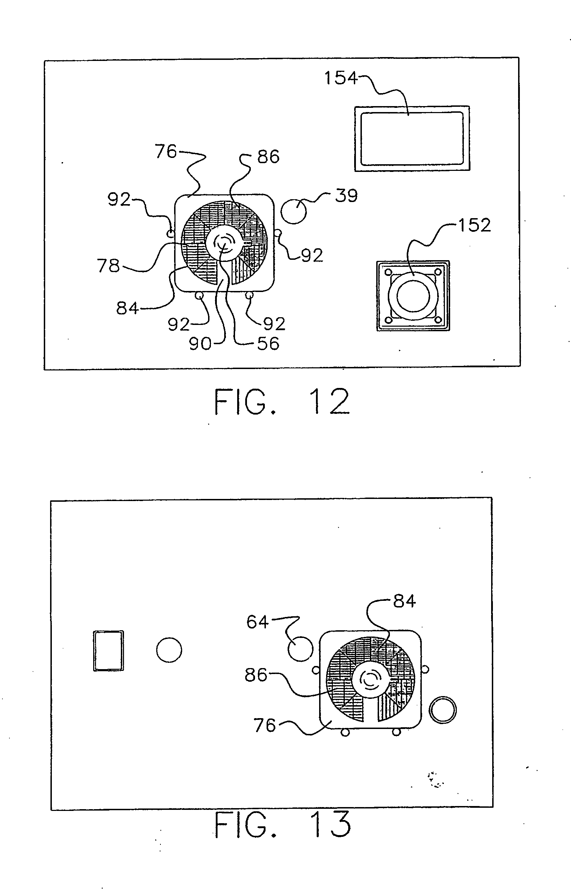

Fig. 12 is a front side plan view of a front panel of a coaxial illuminated laser endoscopic probe and active numerical aperture control apparatus housing showing the first output, illumination level control knob, photoxicity risk card, and laser power meter display and sensor.
Fig. 13 is a right side plan view of the right panel of the coaxial illuminated laser endoscopic probe and active numerical aperture control apparatus housing showing a second output, illumination level control knob, laser connector, power and laser switches, and photoxicity risk card.

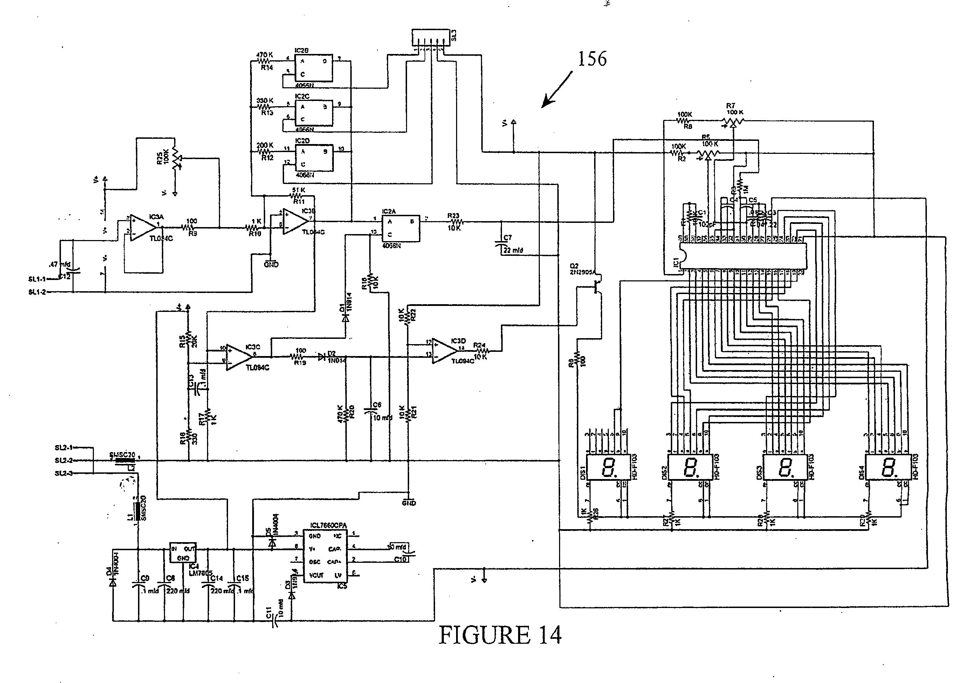

Fig. 14 is an electronic schematic diagram of the laser power meter circuitry.

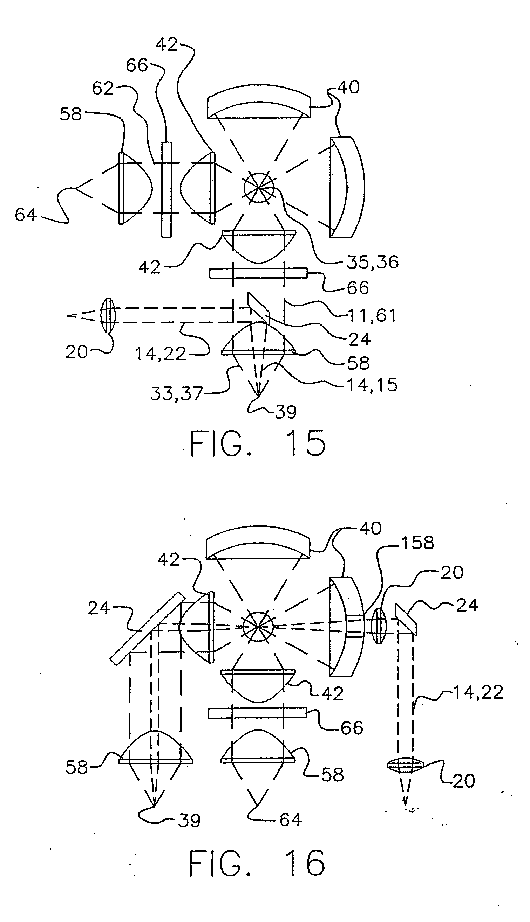

Fig. 15 is an optical schematic diagram of the coaxial illuminated laser endoscopic probe and active numerical aperture control apparatus showing laser and illumination rays, reflectors, mirrors, and lenses.
Fig. 16 is an optical schematic diagram of an alternate embodiment of the coaxial illuminated laser endoscopic probe and active numerical aperture control apparatus showing laser and illumination rays, reflectors, mirrors, and lenses.

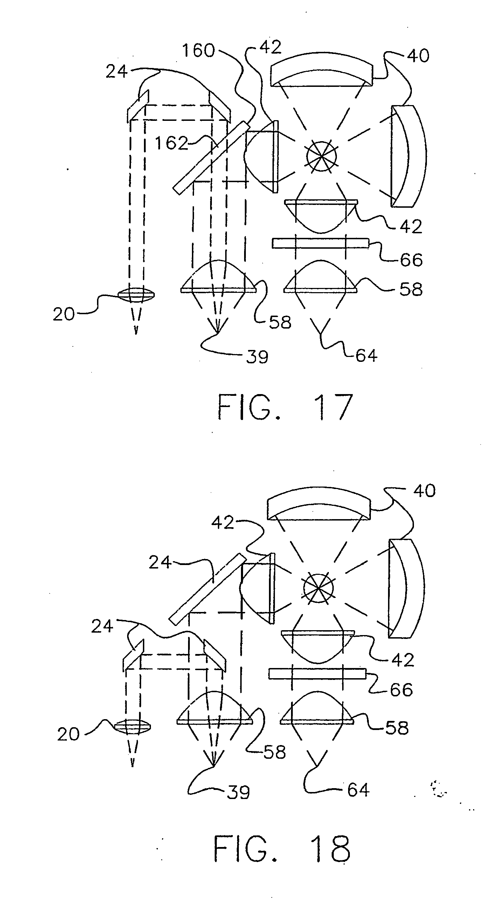

Fig. 17 is an optical schematic diagram of a further alternate embodiment of the coaxial illuminated laser endoscopic probe and active numerical aperture control apparatus showing laser and illumination rays, reflectors, mirrors, and lenses.
Fig. 18 is an optical schematic diagram of another alternate embodiment of the coaxial illuminated laser endoscopic probe and active numerical aperture control apparatus showing laser and illumination rays, reflectors, mirrors, and lenses.

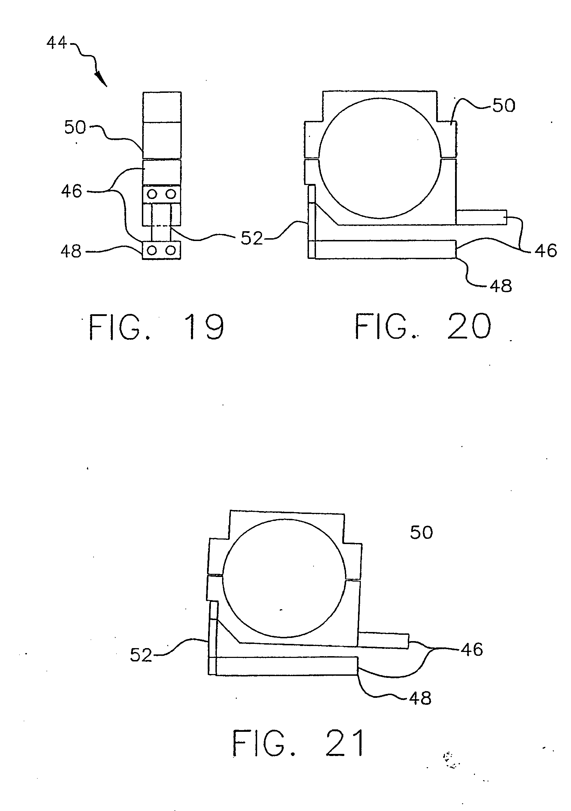

Fig. 19 shows a left side plan view of the first lens mount.
Fig. 20 shows a front side plan view of the first lens mount at a full intensity position
Fig. 21 shows a front side plan view of the first lens mount at a dimmed intensity position.

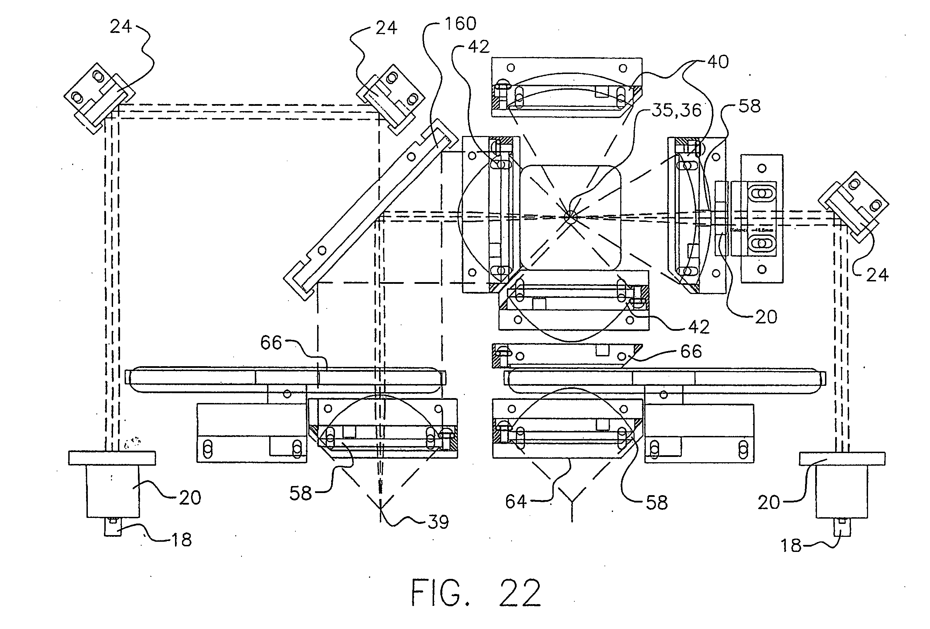

Fig. 22 shows a top plan view of an implementation of the alternate embodiments of the coaxial illuminated laser endoscopic probe and active numerical aperture control apparatus as shown in the optical schematics of Figs. 16 & 17 showing illumination and laser light paths without the phototoxicity card, power meter, and ferrule connectors.

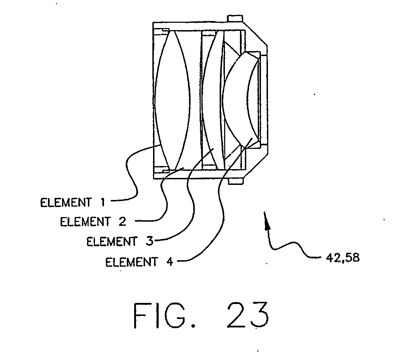

Fig. 23 shows a side plan half cross sectional view of the preferred embodiment of the first and second lenses which correct for color, spherical aberration, and coma and have a back focus 20mm from the apex of the last element and a numerical aperture of .5.

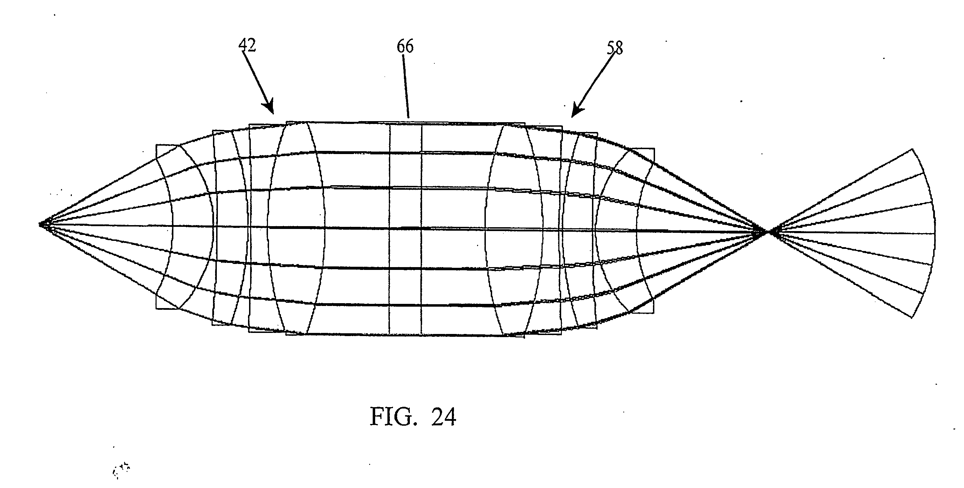

Fig. 24 shows an optical schematic of the first lens set, collimated space, dichroic hot mirror filter, and second lens set with illumination light path rays shown.

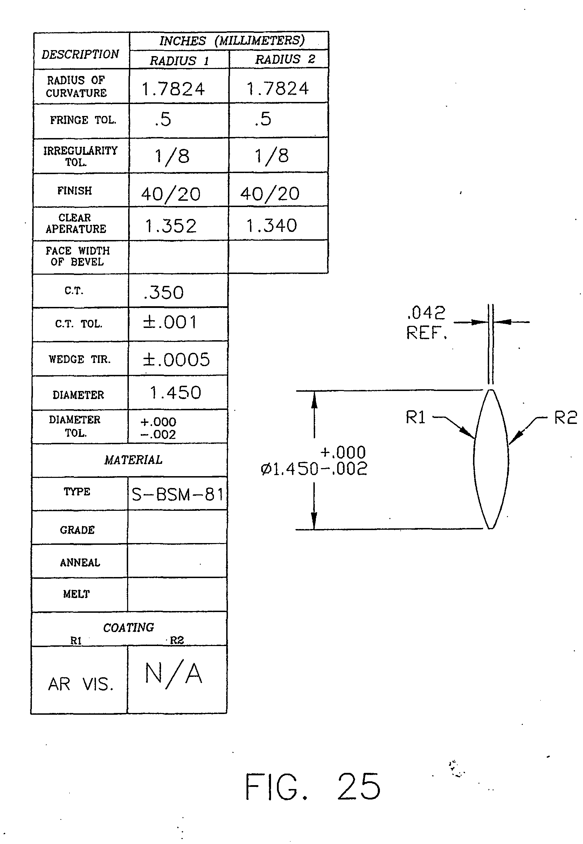

Fig. 25 shows a detailed side plan half cross sectional view with dimensional attributes of the preferred embodiment of element 1 of the lens shown in Fig. 23.

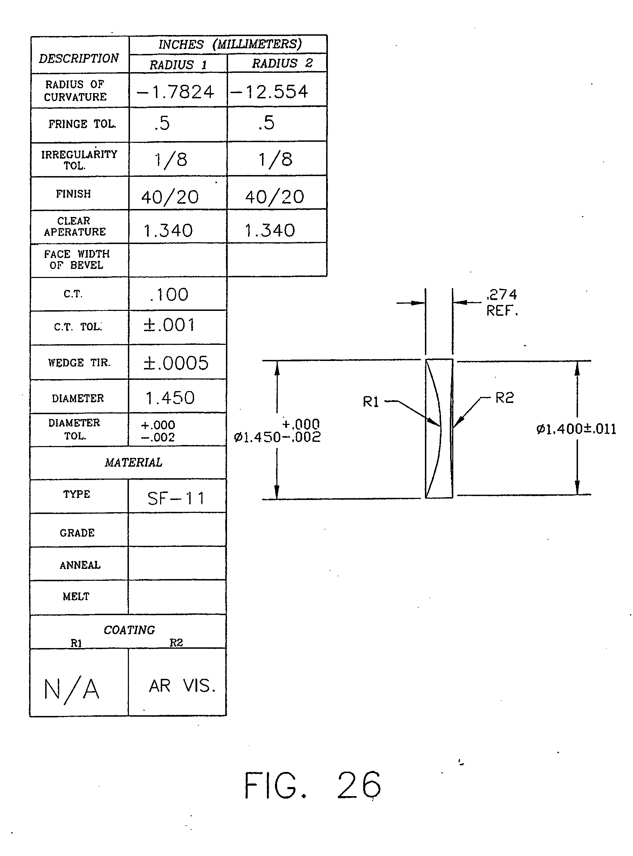

Fig. 26 shows a detailed side plan half cross sectional view with dimensional attributes of the preferred embodiment of element 2 of the lens shown in Fig. 23.

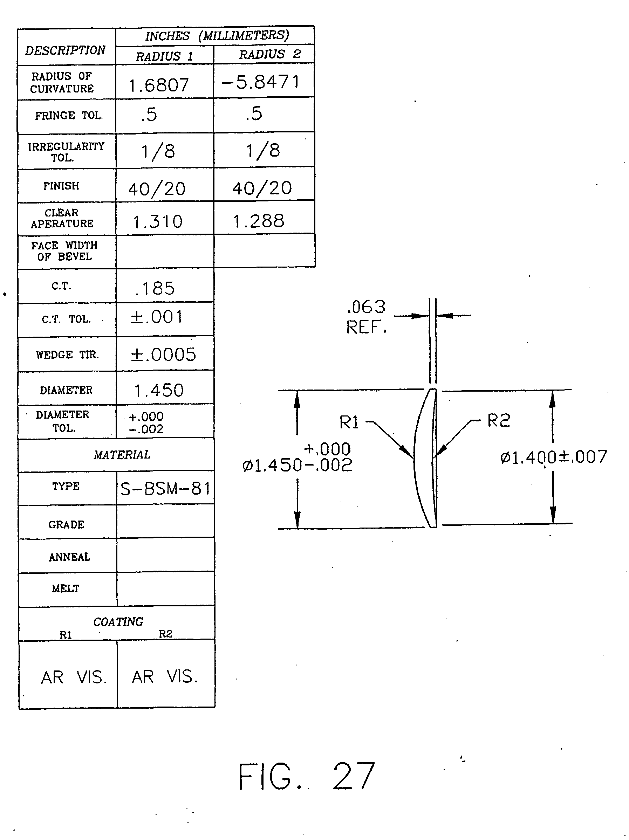

Fig. 27 shows a detailed side plan half cross sectional view with dimensional attributes of the preferred embodiment of element 3 of the lens shown in Fig. 23.

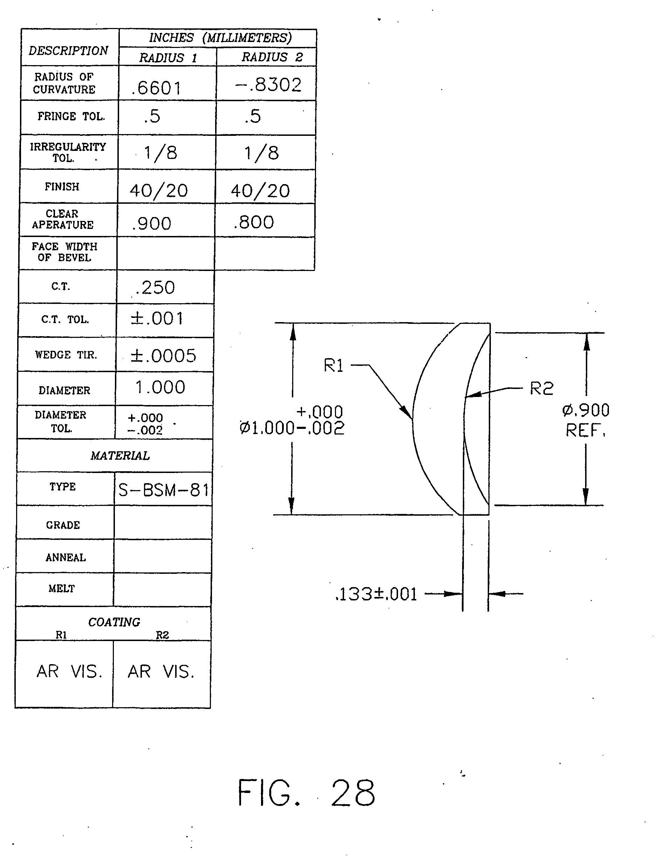

Fig. 28 shows a detailed side plan half cross sectional view with dimensional attributes of the preferred embodiment of element 4 of the lens shown in Fig. 23.

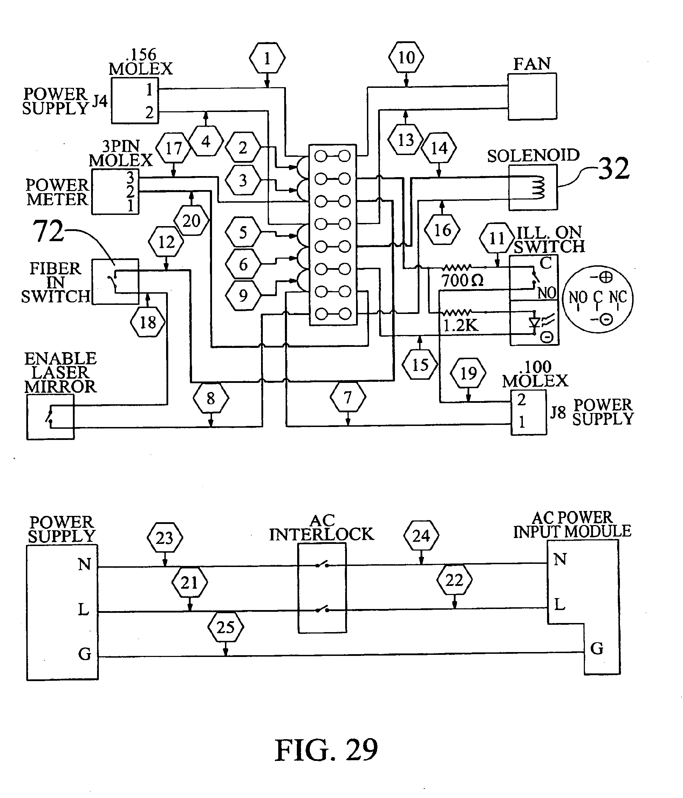

Fig. 29 shows an electrical schematic diagram of the coaxial illuminated laser endoscopic probe and active numerical aperture control apparatus.

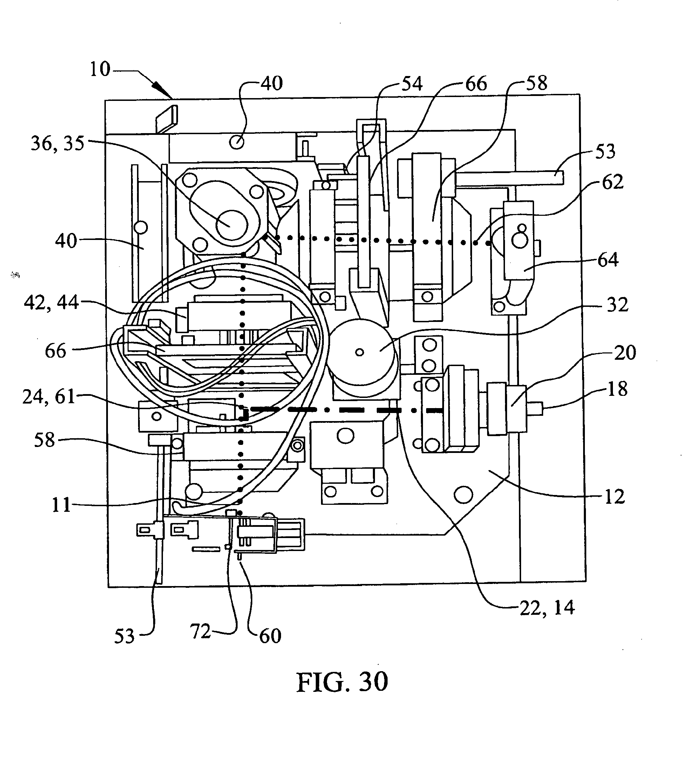

Fig. 30 shows a top perspective view in black and white photographic form of a preferred embodiment of the coaxial illuminated laser endoscopic probe and active numerical aperture control apparatus showing illumination and laser light paths without the phototoxicity card, power meter, and ferrule connectors.

### DETAILED DESCRIPTION

Referring now to the drawings, there is shown in the Figures embodiments of the coaxial illuminated laser endoscopic probe and active numerical aperture control apparatus 10 also herein described as an illumination and laser source 10. There is provided a device 10 for providing non-coherent illumination light 11, 62 and coherent laser treatment light 14 through a single optical fiber 60 of the size typically used for laser treatment only in a safe, effective, and user friendly manner. The apparatus is especially suited for use during ophthalmic surgery.

In a preferred embodiment a 75 watt xenon arc lamp **36** is used for its high luminance illumination (light density), greater than 6000 °K color temperature, and greater than 95 color rendering index. A unique and useful feature is the very high luminance and small size plasma ball formed on the end of the lamp **36** cathode. If imaged correctly the plasma ball is bright enough to provide the required illumination input to a small fiber such as that used for laser treatment. The xenon arc lamp **36** further provides an extremely small point light source which allows for a smaller output illumination beam **37** diameter. Unique to the present lamp source is a mount **38** which allows for replacement of the lamp **36** and yet retains the location of the plasma ball of said source **36** precisely at a predetermined location within the optical center 35 of the apparatus.

A classic spherical reflector **40** and two lens **42, 58** light collection layout is utilized rather than other lower part count layouts, such as using an elliptical reflector or a combination of a parabolic reflector and lens. This technique allows maximum collection efficiency with a minimum of geometric aberration. The lamp **36** is located at the geometrical center **35** of the reflector **40** and at the focus (focal point) of the first lens **42.** Light that is incident on the reflector **40** is reflected back to the lamp **36.** This forms an upside down or inverted image of the source **36** coincidental to the source **36.** The first lens **42** collimates light from the source **36** and the upside down or inverted image. The second lens **58** is located coaxial to the first lens **42** and focuses the light at its focal point. The output optical fiber **60** is located at the focal point of the second lens **58.** The aforesaid reflectors **40** are preferably spherical rather than parabolic in order to reflect illumination light in the same form as sourced from the arc lamp **36.**

Best form lenses **42, 58** (plano convex aspheric, facing each other) are used. It was discovered that chromatic aberrations, caused by the lenses, gave the output of the optical fiber **39, 60** either a yellow or blue cast. This is not a problem with other ophthalmic sources because the source is many times larger than the output optical fiber. A color corrected "f l" or possibly .5 numerical aperture lens set **42, 58** consisting of four elements was designed to be utilized for each lens. Each of the elements is coated with a MgF (magnesium fluoride) anti-reflective coating to minimize light losses, with other anti-reflective coatings or layers also utilizable. Use of the achromatic lens sets allows a high fidelity image of the illumination source **36** to be focused onto the end of the optical fiber **60, 64.** That is, the multi-element lenses allow for a minimum of chromatic aberration. The aforesaid four element lens set is shown and specifically described in the Figures.

An additional separate illumination path **62** is possible. A 0.5 system numerical aperture or "f 1" lens is the greatest practical because of limitations to the numerical aperture of available optical fibers. This equates to 60 degrees full angle. When the spherical reflector **40** is considered, an additional 60 degrees is provided from the total of 360 degrees available. Consideration of the vertical rotation around the source **36** is impractical because of shadows caused by the lamp **36** electrodes. A total of 240 degrees of horizontal rotation around the lamp 36 are left unaccounted for. Allowing for optics mounts **44** does account for some additional amount. However, at least half of the illumination output is available. This leaves room for a second light path **62** located orthogonal to the first path **11** along with the second fiber output **64.** No other conventional illumination light source incorporates multiple light paths from a single lamp, that is two independent collection systems for illumination light. The independent nature of the two paths **11, 62** allow different filtering and intensity control settings to the two outputs **39, 41.**

Output dimming of the present art illumination system is accomplished by steering the first (collimating) or penultimate lens **42** in a fashion that does not change the lens **42** numerical aperture or introduce shadow artifacts into the beam **37.** The lens set mount **44** has two halves **46** and a flat spring **52.** The first part **48** is attached to the optics bench **12,** the second part **50** holds the lens set **42,** and the spring **52** connects the two **48, 50** together on one side. Pressure on the lens mount second part **50** causes the spring **52** to deflect and the lens **42** to move in a direction generally perpendicular to the optical axis. This results in motion or movement of the image across the face of the optical fiber **60, 64** whereby the peak illumination of the beam **37** is not centered on the optical fiber **60, 64** face during dimming. Due to the aforesaid, the reduction of the output light from the fiber **60, 64** without affecting the color (i.e. color temperature) or aperture of the output is achieved. In a preferred embodiment a shaft mounted cam **54** applies the pressure to the lens mount second part **50** and spring **52.** A control knob **56** is attached to the other end of the shaft **53** and allows the user to select the desired illumination level by rotating the knob **56.** This method is capable of providing at least 95% reduction in output illumination intensity. In a preferred embodiment, a shutter **57** is mounted upon the shaft **53** and is rotated across the illumination beam **37** in order to fully attenuate the output illumination intensity upon full rotation of said knob **56.** Alternative embodiments may utilize other methods, including but not limited to electric or electronic drives, to rotate said shaft **53** instead of said knob **56.**

A dichroic "hot" mirror filter **66** is placed in the collimated space **61** between the illumination lenses **42, 58.** This provides both UV and IR filtering of the light. Brackets are attached to the hot mirror **66** mount to provide a means for additional user selectable filters. Positioning of the filters is critical because this is the only area where the light **11** is generally normal to the filter surface. Location of the filter **66** on the other sides of the lenses would cause the light to have many undesirable incidence angles (between 0 and 30 degrees). Variation in the incidence angle causes dichroic reflectors or filters to have a shift in their affect. If absorption filters are used, placement outside the collimated space **61** will cause an increase in reflective losses and heating problems.

The output optical fiber connector **98** is uniquely configured to provide the precise positioning required while reducing cost. A precise connector or mating end **116** is combined with an integral retention thread **130** to reduce parts cost and assembly time. An optional groove or recess **148** is placed on a second version of the connector to provide for sensing the difference between illumination only and laser compatible output fibers. Placement of a smooth diameter connector **74** into the output activates a switch **72** which will allow the laser power to be mixed. Either the lack of a connector **98** or the groove or recess **148** under the switch **72** will cause the switch **72** to not activate and the laser power will not be mixed in.

Regarding mixing of laser treatment energy or light **14,** laser light **14** is delivered to the system via a preferably 50 µm optical fiber **16** or equivalent. The connector **18** on the laser end is configured to be compatible with the laser and to provide the necessary interface to signal to the laser that a fiber is connected. The laser and light source **10** end preferably uses an SMA 905 connector or equivalent to allow repeatable connections of the laser delivery fiber **16.** Laser light **14** exiting the delivery fiber is preferably collimated using a 16 mm focal length achromatic lens or equivalent **20,** i.e. laser collimating lens, which can also be utilized to focus the collimated laser beam **22.** The position of the fiber **16** is adjusted to be at the focal point of the lens **20.** The input laser connector **18** and collimating lens **20** are located so that the collimated beam **22** is orthogonal to and intersects the center of the illumination axis **11** between the illumination lens sets **42, 58** (the collimated area for illumination light). If all safety requirements are met (i.e. laser output compatible fiber inserted and selection switch for laser output activated) a steering mirror **24** reflects the collimated laser light **22** into the center of the illumination axis **11.** The steering mirror **24** is a first surface plano that is positioned at 45 degrees to the laser light **14** and is located in the center of the illumination axis **11** (when laser mode is active). The thickness of the mirror **24** is shaped to appear as a circle when viewed along the illumination axis. Due to the 45 degree surface orientation, the shaping causes the mirror **24** surface to appear elliptical when viewed from a normal angle. The size of the mirror **24** is chosen to be minimally larger that the collimated laser beam **22.** Placement of the steering mirror **24** in the center of the illumination axis **11** causes the light rays that would normally be there to be blocked and a shadow to appear in the center of the output light cone. The second illumination lens **58** focuses the laser light **14** reflected by the steering mirror **24** onto the end of the output fiber **60, 64.** Because the length of the output optical fiber strand is relatively short, the incidence angle of light entering the input end is very nearly the same angle on the output end. This results in the output of the fiber strand having a cone of white light with a shadow in the center nearly filled with the laser aiming beam (treatment beam during treatment). That is, the laser provides an aiming beam, typically red, when not fully activated for treatment and a treatment beam, typically green, when fully activated. Without the shadow caused by the steering mirror **24** the aiming beam would be entirely washed out or imperceptible except at very low illumination levels.

Alternate embodiments may utilize more than one steering mirror **24** or place the steering mirror **24** outside of the illumination axis or illumination light path **11** and direct the laser light **14** through an aperture **158** in said spherical reflector **40** and thereafter through the arc lamp **36** plasma ball or through a dichroic reflector **160** or a reflector having an aperture **162.** All of the aforesaid alternate embodiments place the laser light **14** within the collimated space **61** and utilize the second lens **58** for focus upon the output optical fiber **60.** Moreover, all of the aforesaid alternate embodiments provide for a second light path **62** output as seen in the Figures.

The laser steering mirror **24** is mechanically mounted on a thin post **28** that holds it in place while minimizing the loss of illumination light **11.** The post **28** is mechanically connected to a bracket **30** which is connected to a solenoid **32.** The solenoid **32** causes the bracket **30** and also the steering mirror **24** to move into one of two positions. Position one is outside the collimated illumination and laser light. This position is used for no laser delivery and allows the illumination path to operate unaffected. Position two is with the steering mirror **24** located to reflect the laser light into the illumination path **11.** Motion of the solenoid **32** and bracket **30** are controlled by a precision ball slide **34.** Use of the slide **34** insures repeatable positioning of the mirror **24.**

Further alternate embodiments of the apparatus **10** may utilize parabolic reflectors instead of spherical reflectors in order to collimate the illumination source **36.** This technique would eliminate the need for the first collimating lens **42** and allow transmission of the laser beam **22** through an aperture within the parabolic reflector or via a steering mirror **24** within the collimated space **61.** Still further alternate embodiments may utilize an elliptical reflector having two focal points whereby the illumination source **36** is placed at the first focal point and the output fiber **60** is placed at the second focal point with the laser beam **22** introduced through an aperture within the elliptical reflector or via a steering mirror **24** between the illumination source **36** and the output fiber **60.** This latter alternate embodiment requires focusing the laser beam **22** onto the output fiber **60** via a lens placed within the laser beam path **22** prior to the output fiber **60** yet allows elimination of both the first collimating lens **42** and the second focusing lens **58.**

Some of the variables which determine the phototoxicity risk level during vitreoretinal surgery include the spectral and power characteristics of the light source used, the type and size of the endoilluminator probe, the length or duration of the surgical procedure, and the area (size) of the illuminated tissues. In each case the surgeon must make a risk-benefit judgement about the intensity of light to be used. Use of insufficient intensity may result in inadequate visualization and adverse effects more serious than a retinal photic injury. Currently, the calculation of the exposure time required to reach a point of injury is a tedious chore involving the numerical integration of the spectral power density function of the light source **36** with a hazard function (see ISO 15752), and specific knowledge of the surgical illumination area and endoilluminator characteristics.

In a preferred embodiment, an inexpensive photoxicity risk card **76** is removably attached to the control panel of the surgical illumination and laser light source **10**. Preferably, the card **76** is attached in close proximity to the light intensity control knob **56** in order to show the relationship between the output intensity of the light source and the likelihood of photic injury. The card **76** is preferably included with each endoilluminator instrument, i.e. optical fiber, that is calibrated to represent the phototoxic performance of that instrument type when used with a particular type of light source. The graphical representation **78** on the card **76** acts as a guide for adjustment of the output intensity of the source **10** in relationship to an accepted standard, that is such as the "Millennium" from Bausch and Lomb®. In this way the spectral and power characteristics of the various elements involved in delivering light to the eye are integrated into a single and easily manageable variable. This greatly reduces the complexity of judging the best intensity to use in a given situation. Alternative embodiment graphical representations **78** could present other information regarding the light output such as lumen output (a unit that is weighted by the photopic response of the eye). Other representations could present threshold information when used with special dyes or colored light filters.

A preferred embodiment comprises a card **76** that is die-cut from white chipboard stock that is approximately the weight of a business card. The shape of the card **76** is generally square with a slot **90** removed from one side to enable the card **76** to be placed behind the intensity control knob **56** of the illumination and laser source **10** while providing clearance for the control shaft **53** which is turned by said knob **56.** In a preferred embodiment, four location pins **92** are attached to the front panel of the illumination and laser source **10** enclosure. The pins **92** provide boundaries for card **76** location and tend to inhibit rotation of the card **76** with the control knob.

In a preferred embodiment, onto the face of the card is printed a circular shaped scale **84** that has different color bands **86** representing the phototoxicity risk at a given intensity level, for example green, yellow, and red. The control knob **56** has an indication line that points to the current output intensity level and concurrent phototoxicity risk associated with the probe being used. The card **76** indicates output intensity at the optical fiber output. The card **76** is meant to be disposed of after a single use and replaced with a new one provided with each optical fiber instrument. In this manner the output of the light source **10** is recalibrated each time it is used. The calibrated unit type may vary with different instrument styles to provide the surgeon with the most pertinent information possible.

As aforesaid the card **76** provides a known point of reference relative to the prior art illumination devices. For example, if the surgeon maintains the knob **56** indicator line within the green color band, he or she will understand that the light intensity output is within the safe intensity of the prior art illuminators such as the "Millennium" from Bausch and Lomb®. This control phenomena is especially useful when utilizing more powerful illumination sources **10** such as described herein. That is, the surgeon must have a prior art point of reference when utilizing more powerful and modem illumination systems. The art of the present Several bands indicate phototoxicity levels or light intensity levels directly to the surgeon.

The manufacturer of the optical fiber is able to provide a phototoxicity risk card **76** which accounts for attenuation and spectral absorption within the optical fiber provided with said card **76.** Thus for example, if an optical fiber is highly attenuating, the card may indicate that the surgeon must turn the intensity control knob **56** to a higher level in order to obtain an equivalency to one or more of the aforesaid prior art illuminators or to achieve a desired photo-illumination output.

The art of the present invention also comprises a ferrule or connector **98** having an internal bore **102,** preferably stepped **104,** which is substantially parallel with the lengthwise axis **100** of the ferrule body **98.** The aforesaid bore **102** allows for placement and bonding or potting of an optical fiber within and through said ferrule body **98.** Externally, said ferrule body **98** is also stepped **112, 148** in a unique form in order to optimally function as described herein.

In a preferred embodiment, the ferrule body **98** has an external end **114** and a mating end **116** and externally comprises a substantially cylindrical head **118** of a first diameter **120** having a first end **122** and a second end **123,** said first end **122** co-located with said external end **114.** Said ferrule body **98** further externally comprises a lip **124** of greater diameter than said head **118** and having a first side **126** and a second side **128** with said first side **126** mounted with said second end **123** of said head **118.** A threaded portion **130** of preferably smaller diameter than said head **118** is attached with and extends from said lip **124** second side **128.** In a preferred embodiment, said threaded portion **130** first comprises an 8-32 UNC thread with a first end **132** and second end **134,** said first end **132** connected with said second side **128** of said lip **124.** Also in a preferred embodiment, said threaded portion **130** has a groove **136** of approximately 0.76 mm (.030 inch) at said first end **132** with approximately 2.29 mm (.090 inch) of said thread 130 thereafter following and another approximately 0.76 mm (.030 inch) groove **136** following said thread **130** at said second end **134.** Externally the ferrule body **98** also has an alignment barrel **138** having a first **140** and second **142** end following said threaded portion **130,** said first end **140** attached with said threaded portion **130.** The second end **142** of said alignment barrel **138** is co-located with said mating end **116** of said ferrule body **98.** Also, said second end **142** of said alignment barrel **138** contains an orifice **144** of substantially equivalent or slightly greater diameter as the optical fiber mounted within said stepped bore **102.** Said orifice **144** is interconnected with said internal stepped bore **102.** Said orifice is approximately 0.28 mm (.011 inch) in diameter and 0.64 mm (.025 inch) in length. Also in a preferred embodiment, said alignment barrel **138** has a chamfer **146** at the circumference of said second end **142.** Preferably said chamfer **146** is of approximately 45 degree angle and 0.38 mm (.015 inch) in length. Alternative embodiments may utilize chamfers of different angles or shapes or forego use of a chamfer altogether.

The alignment barrel **138** of the present art is uniquely shaped within the embodiments to indicate whether laser light or illumination light should be applied to the optical fiber. In a preferred embodiment of the laser ferrule which is not an embodiment of the invention, the alignment barrel is of uniform diameter, approximately .118 diameter, which indicates to the source 36 that laser light or energy is desired. In the embodiment of the invention the alignment barrel contains a recess **148** located approximately 1.9 mm (.075 inch) from said barrel **138** second end **142** and extending approximately 6.8 mm (.268 inch) from said second end **142.** When utilized, the illumination and laser source **10** detects this recess and determines that illumination light and not laser light is desired. Further alternative embodiments may utilize the aforesaid recess **148** embodiment for laser light and the uniform barrel diameter for illumination light.

Internally said stepped bore **102** first comprises a first larger bore substantially within said head portion which is of approximately 2.49 mm (.098 inch) diameter and extends substantially the length of said head. A second intermediate bore of approximately 1.6 mm (.063 inch) diameter extends from said first larger bore to said orifice **144** within said threaded portion **130** and said alignment barrel **138.** Also in a preferred embodiment, the orifice **144** length is approximately 0.635 mm (.025 inch). Alternative embodiments may utilize first and second bores and orifices having a plurality of diameter and length sizes provided that the diameter portions are smaller than the ferrule external portions within which each is located.

When assembled with an optical fiber, the optical fiber extends through said bore **102** and orifice **144** and terminates substantially flush with said ferrule body **98** mating end **116** or second end **142** of said alignment barrel **138.** Preferably said optical fiber is held within said bore **102** via potting or adhesive compounds surrounding said fiber and attaching with said bore **102** of the ferrule **98.**

In a preferred embodiment, the external head **118** diameter is approximately 5.94 mm (.234 inch) with a length of approximately 9.53 mm (.375 inch). The lip **124** external diameter is approximately 7.92 mm (.312 inch) with a thickness of approximately 0.635 mm (.025 inch). Also, said alignment barrel **138** is approximately 3 mm (.118 inch) in diameter and 9.65 mm (.380 inch) in length.

Where provided, dimensions, geometrical attributes, and thread sizes are for preferred embodiment informational and enablement purposes. Alternative embodiments may utilize a plurality of variations of the aforesaid without departing from the scope of the present invention. This is especially true as relating to said head **118,** lip **124,** and threaded portion **130.** Said lip **124** may be integrated as part of the head **118** or removed completely. Also, the position, location, and type of threaded portion **130** may vary. Said threaded portion **130** may not utilize said grooves **136,** utilize grooves of a shorter or longer length, or have said head **118** and lip **124** diameters sized substantially the same as or smaller than the outside diameter of said threads **130.** The art of the present invention may be manufactured from a plurality of materials, including but not limited to metals, plastics, ceramics, or composites.

A laser power meter **150** has a sensor **152,** a power display **154,** and associated control circuitry **156.** The power meter **150** allows a surgeon to place the endoscopic fiber optic probe onto said sensor **152,** energize the laser through the illumination and laser source **10** and measure the laser power output as seen on said display **154.** Inclusion of the aforesaid is especially useful due to variations in optical fibers or to account for attenuation through the illumination and laser source **10.** By utilizing the power meter **150,** the surgeon has complete knowledge of the laser power transmitted to the surgical site. Alternative embodiments may utilize said power meter **150** for measurement of the output illumination **37** intensity as well as the laser light **14** power.

In operation, the surgeon connects a laser light source via optical fiber to the input laser connector **18** on the apparatus **10.** The surgeon thereafter connects a ferrule connector **98** with an integral optical fiber connected with an endoscopic probe at the first output **39** or for illumination only at said second output **64.** If said ferrule connector **98** at said first output **39** does not have the aforedescribed recess **148,** the apparatus **10** will allow the steering mirror **24** to position within the illumination light path **11** and further allow transmission of laser light. If the surgeon desires to measure laser power output, he or she places the output end of the endoscopic probe onto said sensor **152** and upon full laser activation, reads the laser power output on the display **154.** If the apparatus **10** is powered, the surgeon proceeds to illuminate the tissues of concern with a cone of white illumination light having a shadow where the laser beam will be placed and a typically red laser aiming beam within said shadow. Upon full activation of laser power, a typically green treatment laser beam replaces said typically red aiming beam to treat the tissues of concern. All of the aforesaid illumination and treatment may be achieved with a single incision and through a single optical fiber of smaller diameter than prior art sources.

Those skilled in the art will appreciate that a photon illumination and laser ferrule connector **98** has also been shown and described. Said ferrule **98** is especially useful for quick and positive connection of an optical fiber to a laser or illumination source **10** as herein described and further allows said source **10** to distinguish the optical fiber type or use, that is for illumination or medical laser application. The present art device is useful during surgery and especially ophthalmic surgery. Also, those skilled in the art will appreciate the integral inclusion of a laser optical fiber output power meter.

Having described the invention in detail, those skilled in the art will appreciate that modifications may be made of the invention. Therefore, it is not intended that the scope of the invention be limited to the specific embodiments illustrated and described. Rather it is intended that the scope of this invention be determined by the appended claims and their equivalents.

## Claims

1. A ferrule connector for use with a source of surgically useful visible broad spectrum illumination light and a source of laser treatment light comprising:
a ferrule body (98) having an internal bore (102) having an optical fiber (60); and
a head (118); and
a threaded portion (130) attached with and following said head (118); and
an alignment barrel (138) attached with and following said threaded portion (130); and
a mating end (116) opposite said threaded portion (130); and
an orifice (144) in said mating end (116) of substantially equivalent or greater diameter than said optical fiber (60),
**characterized by**
a recess (148) within said alignment barrel (138) for distinguishing said optical fiber (60) for use with the laser treatment light source, the illumination light source (36), or both.

2. The ferrule connector for use with an illumination or laser source as set forth in claim 1, whereby:
said threaded portion (130) is an 8-32 UNC thread.

3. A method of utilizing a ferrule connector for transmitting surgically useful visible broad spectrum illumination light or laser treatment light through a single optical fiber, the ferrule connector comprising:
a ferrule body (98) having an internal bore (102) in which an optical fiber (60) is placed, the ferrule body (98) having an external end (114) and a mating end (116),
a head (118) having a first end (122) and a second end (23), the first end (122) of the head (118) being co-located with the external end (114) of the ferrule body (98); and
an alignment barrel (138) having a first end (140) and a second end (142), the second end (142) of the alignment barrel (138) being co-located with the mating end (116) of the ferrule body (98);
an orifice (144) in said mating end (116) of substantially equivalent or greater diameter than said optical fiber (60); and
a recess (148) within said alignment barrel (138),
**characterized by**
utilizing said recess (148) to distinguish said optical fiber (60) for use with the laser treatment light (14), said illumination light (36), or both.

## Patentansprüche

1. Ferrulenverbinder zur Verwendung mit einer Quelle von chirurgisch verwendbarem sichtbarem Breitband-Beleuchtungslicht und einer Quelle von Laserbehandlungslicht mit
einem Ferrulenkörper (98), der eine Innenbohrung (102) mit einer optischen Faser (60) aufweist, und
einem Kopf (118) und
einem Gewindeabschnitt (130), der mit dem Kopf (118) verbunden ist und auf ihn folgt, und
einem Ausrichtungszylinder (138), der mit dem Gewindeabschnitt (130) verbunden ist und auf ihn folgt; und
einem dem Gewindeabschnitt (130) entgegengesetzten Passende (116) und
einer Öffnung (144) in dem Passende (116), die einen im Wesentlichen äquivalenten oder größeren Durchmesser als die optische Faser hat,
**gekennzeichnet durch**
eine Aussparung (148) in dem Ausrichtungszylinder (138) zur Unterscheidung der optischen Faser (60) für die Verwendung mit der Laserbehandlungslichtquelle, der Beleuchtungslichtquelle (36) oder beiden:

2. Ferrulenverbinder zur Verwendung mit einer Beleuchtungs- oder Laserquelle gemäß Anspruch 1, wobei der Gewindeabschnitt (130) ein 8-32 UNC-Gewinde ist.

3. Verfahren zur Verwendung eines Ferrulenverbinders zur Übertragung von chirurgisch verwendbarem sichtbarem Breitband-Beleuchtungslicht oder Laserbehandlungslicht durch eine einzelne optische Faser, wobei der Ferrulenverbinder
einen Ferrulenkörper (98), der eine Innenbohrung (102) aufweist, in der eine optischen Faser (60) platziert ist, wobei der Ferrulenkörper (98) ein äußeres Ende (114) und ein Passende (116) aufweist,
einen Kopf (118) mit einem ersten Ende (122) und einem zweiten Ende (23), wobei das erste Ende (122) des Kopfes (118) mit dem äußeren Ende (114) des Ferrulenkörpers (98) zusammengelegt ist, und
einen Ausrichtungszylinder (138) mit einem ersten Ende (140) und einem zweiten Ende (142), wobei das zweite Ende (142) des Ausrichtungszylinders (138) mit dem Passende (116) des Ferrulenkörpers (98) zusammengelegt ist,
eine Öffnung (144) in dem Passende (116), die einen im Wesentlichen äquivalenten oder größeren Durchmesser als die optische Faser (60) hat, und
eine Aussparung (148) in dem Ausrichtungszylinder (138) aufweist,
**gekennzeichnet durch**
die Verwendung der Aussparung (148) zur Unterscheidung der optischen Faser (60) für die Verwendung mit dem Laserbehandlungslicht (14), dem Beleuchtungslicht (36) oder beiden.

## Revendications

1. Connecteur de ferrule destiné à être utilisé avec une source de lumière d'éclairage à large spectre visible et une source de lumière de traitement au laser d'utilité chirurgicale, comprenant :
- un corps de ferrule (98) présentant un alésage interne (102) comportant une fibre optique (60) ;
- et une tête (118); et
- une partie filetée (130) fixée à et suivant ladite tête (118) ; et
- un cylindre d'alignement (138) fixé à et suivant ladite partie filetée (130); et
- une extrémité d'accouplement (116) opposée à ladite partie filetée (130); et
- un orifice (144) dans ladite extrémité d'accouplement (116) d'un diamètre sensiblement égal ou supérieur à ladite fibre optique (60),
**caractérisé par**
un évidement (148) à l'intérieur dudit cylindre d'alignement (138) pour distinguer ladite fibre optique (60) pour une utilisation avec la source de lumière de traitement au laser, la source de lumière d'éclairage (36), ou les deux.

2. Connecteur de ferrule destiné à être utilisé avec une source éclairage ou laser selon la revendication 1, dans lequel :
ladite partie filetée (130) est un filet UNC 8-32.

3. Procécé d'utilisation d'un connecteur de ferrule pour transmettre une lumière d'éclairage à large spectre visible ou une lumière de traitement au laser d'utilité chirurgicale à travers une fibre optique unique, le connecteur de ferrule comprenant :
- un corps de ferrule (98) présentant un alésage interne (102) dans lequel est placée une fibre optique (60), le corps de ferrule (98) possédant une extrémité extérieure (114) et une extrémité d'accouplement (116),
- une tête (118) ayant une première extrémité (122) et une seconde extrémité (23), la première extrémité (122) de la tête (118) étant colocalisée avec l'extrémité extérieure (114) du corps de ferrule (98) ; et
- un cylindre d'alignement (138) ayant une première extrémité (140) et une seconde extrémité (142), la seconde extrémité (142) du cylindre d'alignement (138) étant colocaliséé avec l'extrémité d'accouplement (116) du corps de ferrule (98) ;
- un orifice (144) dans ladite extrémité d'accouplement (116) d'un diamètre sensiblement égal ou supérieur à ladite fibre optique (60) ; et
- un évidement (148) à l'intérieur dudit cylindre d'alignement (138),
**caractérisé par**
l'utilisation dudit évidement (148) pour distinguer ladite fibre optique (60) pour une utilisation avec la lumière de traitement au laser (14), ladite lumière d'éclairage (36), ou les deux.
